# EUROPEAN PATENT APPLICATION

(11) **EP 2 404 557 A1**
(43) Date of publication of application: **11.01.2012**
(21) Application number: 10305751.9
(22) Date of filing: 06.07.2010
(51) Int. Cl.: A61B 17/11, A61B 17/06, A61F 2/08, A61F 2/00

(54) **Barbed scaffolds**

(71) Applicant: Tornier, Inc., Edina, MN 55435 (US)
(72) Inventor: Peterson, Dale R., LA JOLLA - USA, CA 92037 (US); Anderson, Justin, MINNETONKA - USA, MN 55345 (US)
(74) Representative: Grand, Guillaume

(57) **Abstract**

A scaffold for tissue reinforcement according to embodiments of the present invention includes a plurality of barbs configured to engage the tissue. Such scaffolds may include braids with barbed fibers in the axial positions, braids with flexible barb strips in the axial positions, weaves with barbed fiber in the warp positions, weaves with flexible barb strips in the weft positions, and cerclages or other structures with barbs on one surface to lock the scaffold upon itself. Such barbs may be configured to substantially prevent sliding in one or more directions, depending upon an orientation of the barbs. Flexible tack strips may be biocompatible and/or resorbable.

## Description

### TECHNICAL FIELD

Embodiments of the present invention relate generally to tendon repair, and more specifically to barbed scaffolding and cerclages.

### BACKGROUND

Tendon, and particularly old, thin, and/or friable tendon, does not hold suture well. Typically, tendon repair is achieved using sutures. Scaffold augmentation is used in a small percentage of cases, and such cases also involve attachment of the scaffold to the tissue using suture. Scaffolds may be used to reinforce thin, weak tendons, but the sutures used to attach such scaffolds to the tissue are often a point of failure, and re-tears are relatively common. Also, arthroscopic delivery of scaffolds is difficult and time-consuming.

### SUMMARY

A subject of the present invention is a scaffold for tissue reinforcement, as defined in appended claim 1. Additional advantageous features of this scaffold are specified in dependent claims 2 to 11.

Furthermore, a scaffold for tissue reinforcement according to embodiments of the present invention includes a plurality of barb strips and a plurality of fibers, wherein the plurality of fibers is braided about the plurality of barb strips to form a braided scaffold, and wherein the plurality of barb strips forms axial components of the braided scaffold.

For the scaffold as defined here above, the plurality of barb strips may comprise an average barb length that is at least as long as a diameter of each of the plurality of fibers.

For the scaffold as defined here above, the average barb length may be between the diameter and twice the diameter.

The scaffold as defined here above may further comprise a top surface and a bottom surface, and the plurality of barb strips may comprise a plurality of barbs, and each of the plurality of barb strips may be oriented within the braided scaffold such that each of the plurality of barbs extends from the bottom surface but not the top surface.

For the scaffold as defined here above, each of the plurality of barbs may extend uni-directionally from the bottom surface.

For the scaffold as defined here above, the plurality of barbs, when engaged with a tissue, may be configured to substantially inhibit sliding of the scaffold with respect to the tissue.

For the scaffold as defined here above, the plurality of barbs, when engaged with the tissue, may be configured to distribute a tension load applied to the scaffold substantially across each of the plurality of barbs in contact with the tissue.

For the scaffold as defined here above, the plurality of barbs may be configured to substantially inhibit sliding of the scaffold with respect to the tissue in one direction.

For the scaffold as defined here above, the plurality of fibers may comprise a first set of fibers and a second set of fibers, the first set of fibers may extend along a first longitudinal direction, the second set of fibers may extend along a second longitudinal direction at an angle to the first longitudinal direction, and the barb strips may extend along a third longitudinal direction at an angle to both the first and second longitudinal directions.

For the scaffold as defined here above, first angle between the first longitudinal direction and the third longitudinal direction may be substantially the same as a second angle between the second longitudinal direction and the third longitudinal direction.

A biocompatible scaffold device according to embodiments of the present invention includes a fibrous area and a barbed area comprising a plurality of barbs, wherein the plurality of barbs is configured to entangle with and attach to the fibrous area when the barbed area is placed against the fibrous area.

For the biocompatible scaffold device as defined here above, the barbed area may be an outer surface of a braided scaffold, the fibrous area may be an inside of the braided scaffold, and the plurality of barbs on the outer surface may by configured to entangle with and attach to the inside when the braided scaffold is inserted through itself.

For the biocompatible scaffold device as defined here above, the braided scaffold may be adapted to form a cerclage when the braided scaffold is inserted through itself.

The biocompatible scaffold device as defined here above may further comprise an inner surface and an outer surface, the barbed area may comprise one or more barb strips on the inner surface, and the fibrous area may comprise at least part of the outer surface.

For the biocompatible scaffold device as defined here above, the plurality of barbs on the one or more barb strips may be configured to permit the outer surface to be inserted within the inner surface along a first direction while substantially preventing release of the outer surface from the plurality of barbs along a second direction opposite to the first direction.

The biocompatible scaffold device as defined here above may be configured to form a cerclage when the outer surface is inserted within the inner surface.

For the biocompatible scaffold device as defined here above, the one or more barb strips may permit tightening of the cerclage and inhibit loosening of the cerclage.

A scaffold for tissue reinforcement according to embodiments of the present invention includes a plurality of barb strips and a plurality of fibers, wherein the plurality of barb strips and the plurality of fibers are woven together into a weave, wherein the plurality of barb strips form weft elements of the weave and the plurality of fibers form warp elements of the weave.

For the scaffold as defined here above, the plurality of fibers may be a plurality of non-barbed fibers.

For the scaffold as defined here above, the plurality of fibers may be a plurality of barbed fibers.

For the scaffold as defined here above, an average length of each barb of each of the plurality of barb strips may be longer than an average diameter of each of the plurality of fibers.

For the scaffold as defined here above, the average length may be between the average diameter and twice the average diameter.

For the scaffold as defined here above, the average length may be between the average diameter and thrice the average diameter.

A scaffold for tissue reinforcement according to embodiments of the present invention includes a plurality of scaffold elements and a plurality of barbed fibers, wherein the plurality of barbed fibers and the plurality of scaffold elements are woven together into a weave, wherein the plurality of scaffold elements form weft elements of the weave and the plurality of barbed fibers form warp elements of the weave.

For the scaffold as defined here above, the plurality of scaffold elements may be a plurality of non-barbed fibers.

For the scaffold as defined here above, the plurality of scaffold elements may be a plurality of barb strips.

For the scaffold as defined here above, an average length of each barb of each of the plurality of barb strips may be longer than an average diameter of each of the plurality of barbed fibers.

For the scaffold as defined here above, each of the plurality of barb strips may be flexible.

For the scaffold as defined here above, each of the plurality of barb strips may comprise a resorbable polymer comprising one or more materials selected from the group consisting of lactides, glycolides, hydroxybutyrates, ε-caprolactone, and dioxanones.

For the scaffold as defined here above, each of the plurality of barb strips may comprise a biocompatible polymer comprising one or more materials selected from the group consisting of polyesters, nylons, polyaramides, silk, polyacetal, polyetherketones, polyurethanes, and polyolefins.

A scaffold for tissue reinforcement according to embodiments of the present invention includes a plurality of barbed fibers and a plurality of other fibers, wherein the plurality of other fibers is braided about the plurality of barbed fibers to form a braided scaffold, and wherein the plurality of barbed fibers forms axial components of the braided scaffold.

A scaffold for tissue reinforcement according to embodiments of the present invention includes a tissue contact surface and an outer surface opposite from the tissue contact surface, and a plurality of barbs extending from the tissue contact surface but not from the outer surface, wherein the plurality of barbs is configured to secure the scaffold to tissue when the tissue contact surface is pressed against the tissue.

For the scaffold as defined here above, the plurality of barbs may extend from the tissue contact surface in an orientation that substantially prevents sliding of the scaffold with respect to the tissue in at least one direction.

For the scaffold as defined here above, the plurality of barbs may extend from the tissue contact surface in an orientation that substantially prevents sliding of the scaffold with respect to the tissue in one direction.

A method for making a tissue reinforcement scaffold according to embodiments of the present invention includes braiding a plurality of fibers about a plurality of barb strips to form a braided scaffold in which the plurality of barb strips forms axial components of the braided scaffold.

A method for making a biocompatible scaffold loop according to embodiments of the present invention includes providing a biocompatible scaffold having a fibrous area and a barbed area, wherein the barbed area comprises a plurality of barbs, and entangling the plurality of barbs with the fibrous area to form a loop by placing the plurality of barbs against the fibrous area.

The method as defined here above may further comprise attaching tissue to bone using the loop.

A method for making a tissue reinforcement scaffold according to embodiments of the present invention includes weaving together a plurality of barb strips with a plurality of fibers to form a woven scaffold, wherein the plurality of barb strips form weft elements of the woven scaffold, and wherein the plurality of fibers form warp elements of the woven scaffold.

For the method as defined here above, the woven scaffold may comprise a top surface and a bottom surface, the method further comprising orienting the plurality of barb strips such that barbs of the plurality of barb strips extend from the bottom surface but do not extend from the top surface.

According to an embodiment of the present invention, a mechanism for loop formation comprises an elongated structure which is biocompatible and is braided or woven and has a first end, wherein the first end is inserted into the elongated structure at an insertion zone, the mechanism comprising a plurality of barbs at the insertion zone which inhibit release of the first end from the elongated structure.

For the mechanism as defined here above, the elongated structure may comprise a second end within the insertion zone, the elongated structure further comprising an inner surface, the plurality of barbs extending inwardly from the inner surface, the first end being inserted annularly inside of the second end, within the inner surface along the insertion zone, and back out through a side wall of the elongated structure, such that the plurality of barbs engages an outer surface of the elongated structure to inhibit release of the first end from the elongated structure.

For the mechanism as defined here above, the first end may comprise a needle adapted to facilitate insertion of the first end into the second end and to pierce the side wall of the elongated structure.

For the mechanism as defined here above, the inner surface may comprise one or more barb strips, the one or more barb strips comprising the plurality of barbs.

For the mechanism as defined here above, each of the plurality of barb strips may comprise a biocompatible polymer comprising one or more materials selected from the group: polyesters, nylons, polyaramides, silk, polyacetal, polyetherketones, polyurethanes, and polyolefins.

For the mechanism as defined here above, each of the plurality of barb strips may comprise a resorbable polymer comprising one or more materials selected from the group: lactides, glycolides, hydroxybutyrates, ε-caprolactone, and dioxanones.

For the mechanism as defined here above, the plurality of barbs may be oriented so as to permit the elongated structure to be pulled through itself at the insertion zone in a first direction, while substantially inhibiting movement of the elongated structure with respect to the insertion zone in a second direction opposite from the first direction.

For the mechanism as defined here above, the plurality of barbs may be oriented so as to permit a cerclage formed by the elongated structure to be tightened but not loosened.

For the mechanism as defined here above, the plurality of barbs may protrude from an outer surface of the elongated structure along at least the insertion zone, the first end being inserted through the elongated structure, the plurality of barbs engaging an inside of the elongated structure to inhibit release of the first end from the elongated structure.

For the mechanism as defined here above, the plurality of barbs may engage with a fibrous area on the inside of the elongated structure so as to permit a cerclage formed by the elongated structure to be tightened but not loosened.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 illustrates a top view of a braid or braided structure.
FIG. 2 illustrates a top view of a weave or woven structure.
FIG. 3 illustrates a side cross-sectional view of a scaffold weave in which the warp fibers are barbed, according to embodiments of the present invention.
FIG. 4 illustrates a side cross-sectional view of a scaffold weave in which the warp fibers are barbed on one inner layer of the scaffold, according to embodiments of the present invention.
FIG. 5 illustrates a top view of a scaffold braid in which the braided fibers are braided about barbed fibers, according to embodiments of the present invention.
FIG. 6 illustrates a side perspective view of a barb strip, according to embodiments of the present invention.
FIG. 7 illustrates a top perspective view of the barb strip of FIG. 6.
FIG. 8 illustrates a side perspective view of the barb strip of FIGS. 6 and 7 as a central member about which fibers are braided, according to embodiments of the present invention.
FIG. 9 illustrates a cerclage mechanism with barb strips, according to embodiments of the present invention.
FIG. 10 illustrates an alternative cerclage mechanism, according to embodiments of the present invention.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

Barbed scaffolds and/or barbed looping mechanisms according to embodiments of the present invention may be used to repair and/or reinforce tissue, including tendons and ligaments, in the ankle, shoulder, knee, and other joints. A barbed scaffold sticks to the tissue (e.g. tendon) rather than needing to be sutured to the tissue via loops or eyelets on the scaffold.

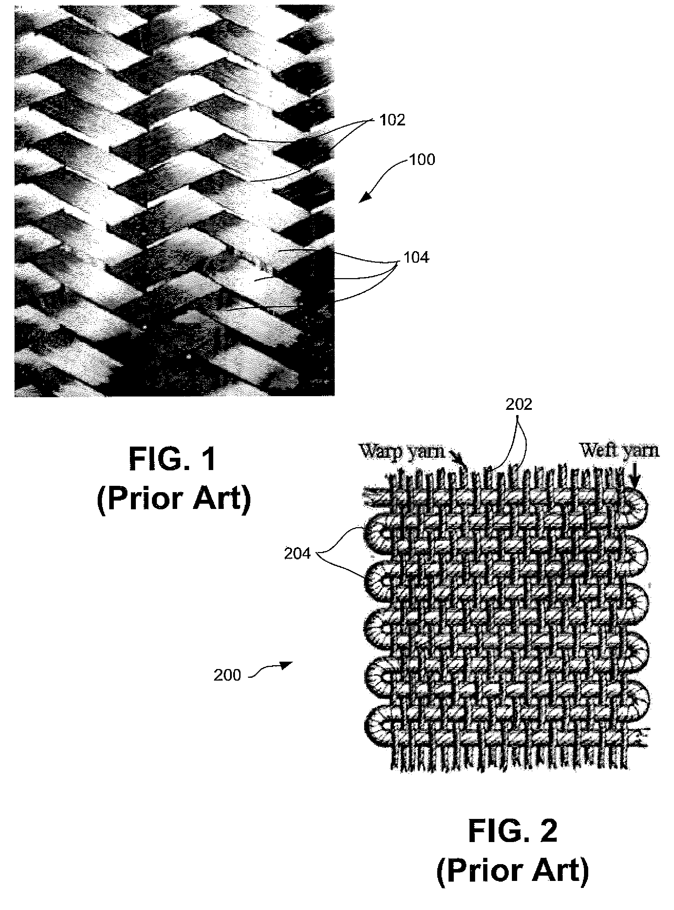

FIG. 1 illustrates a top view of a braid or braided structure 100. The braided structure 100 includes axial components 102 that extend in a substantially straight manner along the structure 100, as well as braid components 104 that are braided about the axial components 102. The axial components 102 are thus encased within the braid components 104.

FIG. 2 illustrates a top view of a weave or woven structure 200. The woven structure 200 includes weft fibers 204 that extend along one direction and warp fibers 202 that are woven above and below the weft fibers 204 along the woven structure 200. The weft fibers 204 may be a continuous looped fiber as illustrated, or each weft fiber 204 may be independent of the adjacent weft fiber 204.

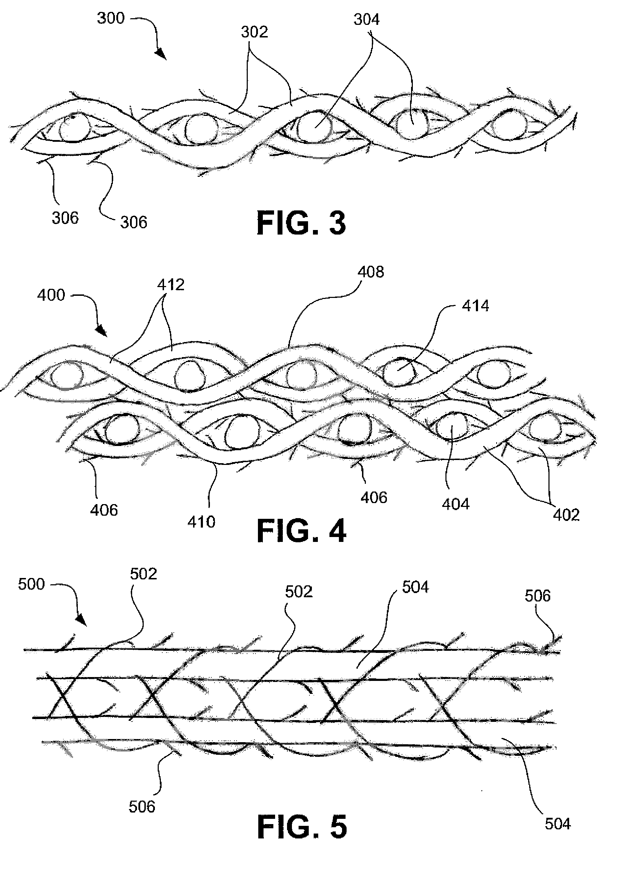

FIG. 3 illustrates a side cross-sectional view of a woven scaffold 300 in which the warp fibers 302 are barbed, according to embodiments of the present invention. The scaffold elements 304 may be placed in the weft position of the weave, and may be non-barbed fibers and/or barb strips (described below), according to embodiments of the present invention.

FIG. 4 illustrates a side cross-sectional view of a woven scaffold 400 in which the warp fibers 402 are barbed on one inner layer 410 of the scaffold 400, according to embodiments of the present invention. Scaffold 400 includes an outer layer 408 and an inner layer 410; the bottom surface of inner layer 410 includes barbs 406 protruding therefrom. Inner layer 410 may be a woven scaffold layer similar to scaffold 300, including barbed warp fibers 402 woven around scaffold elements 404. Outer layer 408 may include warp fibers 412 woven around scaffold elements 414. When warp fibers 412 are non-barbed, the outer layer 408 may be configured to provide a smoother and/or softer surface than inner layer 410, according to embodiments of the present invention. The inner layer 410 of scaffold 400 with barbs 406 may be pressed against or otherwise moved into contact with the tissue that is reinforced, and the barbs 406 may engage the tissue to deter sliding of the scaffold 400 with respect to the tissue and also to deter disengagement of the scaffold 400 from the tissue, according to embodiments of the present invention.

According to embodiments of the present invention, the outer layer 408 and inner layer 410 are two separate layers that are coupled, joined, or bonded, such as, for example, with an adhesive. According to other embodiments of the present invention, certain of the warp fibers 402, 412 (for example non-barbed warp fibers) are woven between the outer layer 408 and inner layer 410 to connect the layers. According to some embodiments of the present invention, one or more additional layers, employing barbed warp fibers and/or non-barbed warp fibers, are interposed between the outer layer 408 and inner layer 410.

FIG. 5 illustrates a top view of a scaffold braid 500 in which the braided fibers 502 are braided about axial components 504, according to embodiments of the present invention. Axial components 504 include a plurality of barbs 506, according to embodiments of the present invention. The braided fibers 502 are braided about the axial components 504 in a manner so as to avoid the barbs 506 and/or to permit the barbs 506 to protrude from the scaffold 500 in its final form, according to embodiments of the present invention.

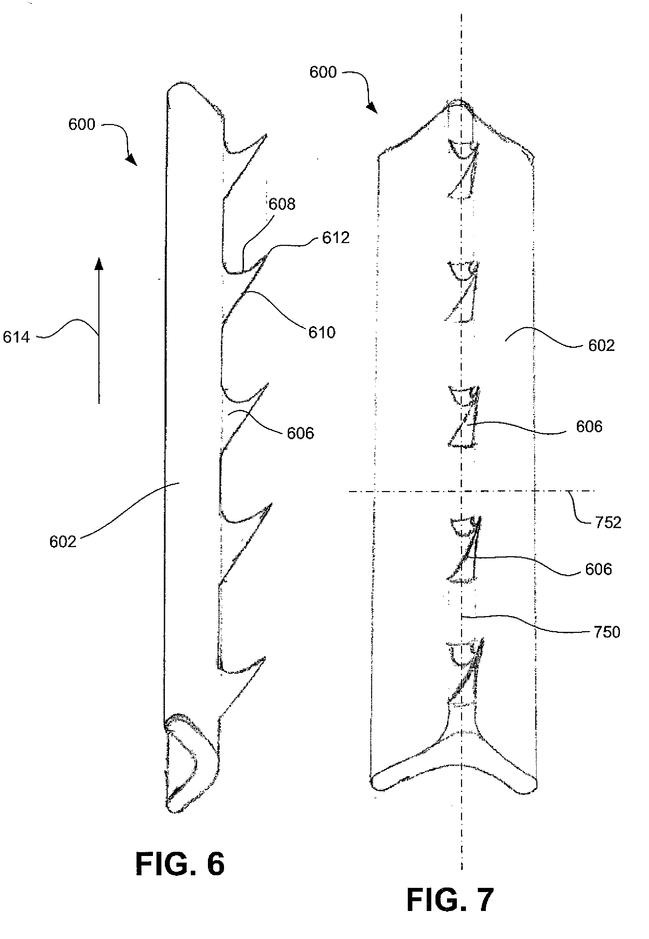

FIG. 6 illustrates a side perspective view of a barb strip 600, according to embodiments of the present invention. FIG. 7 illustrates a top perspective view of the barb strip 600 of FIG. 6. Barb strip 600, which may also be referred to as a tack strip, includes a base portion 602, which may be elongated, and which includes one or more barbs 606 extending therefrom. The barb strip 600 illustrated includes barbs 606 protruding in one direction, although other barb strips may be formed with barbs 606 extending along the same track but at different angular orientations with respect to the longitudinal axis 750 and/or the transverse axis 752, according to embodiments of the present invention. Barbs 606 may also be formed at different areas along the surface of the base 602, according to embodiments of the present invention. As illustrated in FIG. 6, barbs 606 include a point 612, a leading edge 608, and a trailing edge 610, according to embodiments of the present invention. The leading edge 608 may include a trough or indentation, and the trailing edge 610 may include a relatively straight and/or smooth surface, such that when barb strip 600 is engaged with tissue, the barbs 606 substantially hinder or prevent movement of the barb strip 600 with respect to the tissue along a direction indicated by arrow 614, while permitting sliding and/or disengagement of the barb strip 600 from the tissue when the barb strip 600 is moved with respect to the tissue in a direction opposite to arrow 614. Based on the disclosure provided herein, one of ordinary skill in the art will recognize additional barb 606 shapes, orientations, and/or arrangements that will encourage tissue engagement and/or hinder sliding or "walking" of the barb strip 600 with respect to the tissue.

According to embodiments of the present invention, the barbs 606 intended to engage yarns of fibers in a scaffold structure have a length between the diameter of the filaments in the yarn and twice the total yarn diameter. In this way, the barbs 606 are "undercut" enough to engage and trap fibers, yet strong enough to easily bear the applied load through a number of engaged barbs 606, according to embodiments of the present invention. The barbs 606 may be constructed such that their primary failure mode in a medical implant context occurs by deformation rather than by fracture, peeling, or fragmentation. Blends of polyactide and/or polyglycolide with caprolactone or polyhydroxybutyrate may be used to obtain proper barb 606 rigidity and toughness while maintaining biocompatibility and/or resorbability in vivo, according to embodiments of the present invention.

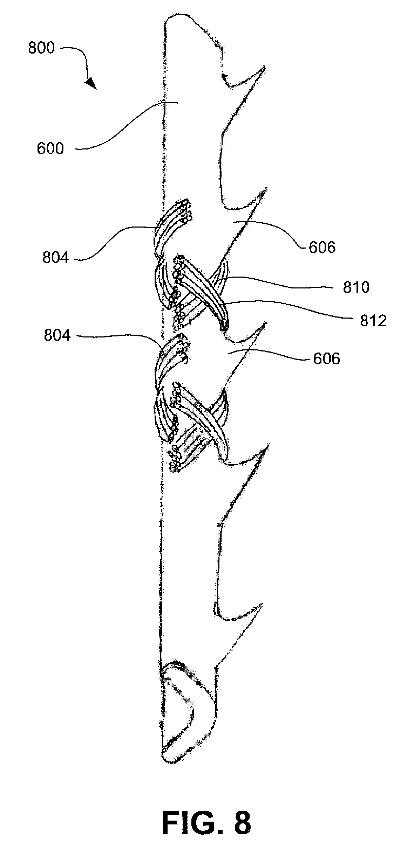

According to embodiments of the present invention, the barb strip 600 may be used as a scaffold element forming a weft component of a woven scaffold. For example, barb strip 600 may be used as scaffold element 304 of woven scaffold 300 (see FIG. 3), or as scaffold elements 404 of inner layer 410 of scaffold 400 (see FIG. 4), according to embodiments of the present invention. According to other embodiments of the present invention, the barb strip 600 may be used as an axial component of a braided scaffold. For example, barb strip 600 may be used as an axial component 504 of a braided scaffold 500 (see FIG. 5), and the braided fibers 502 may be braided so as to avoid the barbs 606, according to embodiments of the present invention. FIG. 8 illustrates a side partial cross section perspective view of the barb strip 600 of FIGS. 6 and 7 as a central member or axial component about which fibers 804 are braided to form a braided scaffold 800, according to embodiments of the present invention. Fibers 804 may be barbed and/or non-barbed, according to embodiments of the present invention. Yarns or fibers may be put into the axial position (e.g. axial component 504) during braiding by feeding yarn (or barb strip 600) in between pairs of braiding yarns 502, according to embodiments of the present invention. The orientation of the barbs may be controlled during such a process as they are fed into the braid, and their geometry may be controlled within the braid by feeding them into selected axial positions. When long barbs 606 are used, the fiber or barb strip 600 may be fed into the core or axial position in the center of the braid, such that when the braiding occurs the barbs 506, 606 extend beyond the braiding elements 502, 804, according to embodiments of the present invention.

According to embodiments of the present invention, the plurality of fibers 804 comprises a first set of fibers 810 and a second set of fibers 812, the first set of fibers 810 extending along a first longitudinal direction, the second set of fibers 812 extending along a second longitudinal direction at an angle to the first longitudinal direction, the barb strips 600 extending along a third longitudinal direction at an angle to both the first and second longitudinal directions (similar to the braiding configuration shown in FIG. 1).

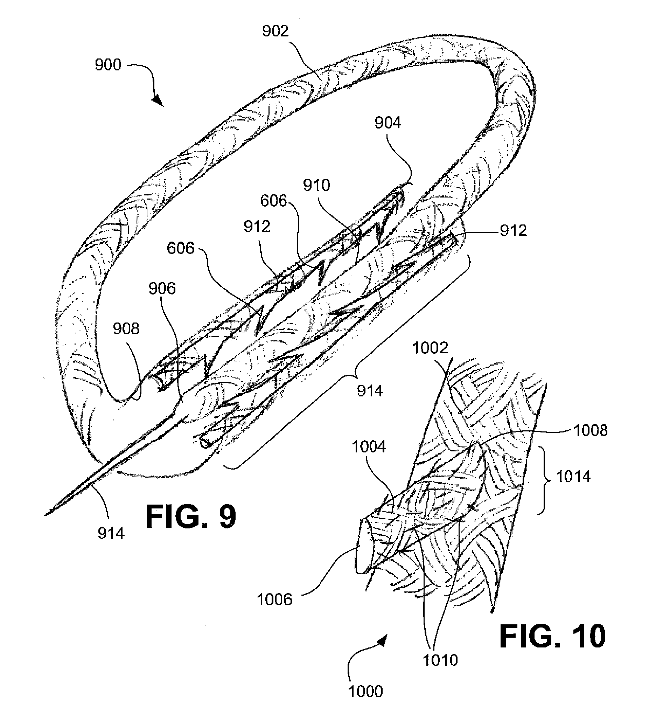

FIG. 9 illustrates a cerclage mechanism 900 with barb strips 912, according to embodiments of the present invention. Cerclage mechanism 900 may also be referred to as a loop formation mechanism. Mechanism 900 includes a main body 902 formed of a woven and/or braided biocompatible and/or bioresorbable material, according to embodiments of the present invention. Main body 902 may be a scaffold similar to scaffolds 300, 400, and/or 500, according to embodiments of the present invention. Main body 902 comprises a back end 904 and a front end 906, as well as an inner surface 908 and an outer surface 910, according to embodiments of the present invention. Main body 902 may be hollow and/or tubular. One or more barb strips 912 may be positioned within the main body 902 against the inner surface 908, with the barbs of the barb strips 912 extending away from the inner diameter 908 (extending inwardly), according to embodiments of the present invention. According to embodiments of the present invention, the barb strips 912 may be placed toward the back end 904 of the main body 902. The main body 902 may be inserted through itself along an insertion zone 914 to form a cerclage or loop, as illustrated in FIG. 9. Insertion of end 906 through end 904 and/or through the barb strips 912 may be facilitated by the formation or insertion of a needle or piercing device 914 at end 906. Needle 914 may also facilitate the formation of a hole through the inner surface 908 for the exit of end 906 through a side wall of the main body 902 after end 906 has been inserted through the barb strips 912, according to embodiments of the present invention. The needle 914 is threaded through the core of the braid 902, past the barb strips 912, and then through the wall of the braid so that the cerclage device 900 can be tightened and the needle 914 and excess braid body material 902 cut off.

As illustrated in FIG. 9, the barb strips 912 and/or barbs thereon may be oriented to permit the main body 902 to be made smaller and/or tightened, while resisting pullout or enlargement of the main body 902. For example, as the end 906 is inserted through barb strips 912 along insertion zone 914 and back through the main body 902 from the inner surface 908 to the outer surface 910, the outer surface 910 contacts the straight or smooth ramped surfaces 610 of the barbs which permit the outer surface 910 to slide over the barbs; however, when the end 906 is pulled in the opposite direction, the sharp ends 612 of the barbs engage the outer surface 910 and hinder further sliding, according to embodiments of the present invention. The one or more barb strips 912 may be located within main body 902 other than at or near end 904, and/or may be located at intervals along the entire main body 902, according to embodiments of the present invention.

FIG. 10 illustrates an alternative cerclage mechanism 1000, according to embodiments of the present invention. Cerclage mechanism includes a main body 1002 formed of a woven and/or braided biocompatible and/or bioresorbable material; main body 1002 has an outer surface 1004 that may be barbed, or at least partially barbed at or near end 1006, according to embodiments of the present invention. The outer surface 1004 includes a plurality of barbs 1010, according to embodiments of the present invention. The main body 1002 may be placed through itself along an insertion zone 1014 to form a loop, by placing end 1006 through an opening 1008 within the main body 1002. This may be achieved, for example, by attaching a needle or piercer to end 1006 and threading it through the main body 1002. The barbs on outer surface 1004 engage with the fibrous area inside of the aperture 1008 to inhibit or substantially prevent release or loosening of the loop, according to embodiments of the present invention. Thus, mechanism 1000 may be formed of woven or braided barbed fibers, according to embodiments of the present invention.

The mechanisms 900 and/or 1000 may be inserted through tissue and attached to bone in order to attach tissue to bone. The cerclage or loop may be formed about the tissue (e.g. through an opening in the tissue), or may be formed through an opening in the tissue as well a bone tunnel, to attach the tissue to the bone, according to embodiments of the present invention. Hence, such devices 900, 1000 may in some cases attach tissue to bone without the use of suture. According to some embodiments of the present invention, a loop mechanism may be formed which employs a combination of one or more of the features described with respect to FIGS. 9 and 10 in order to prevent loop widening and/or pullout.

During use or implantation, the scaffold (e.g. scaffold 300, 400, 500) is positioned over the tissue to be reinforced, and the barbed surface is pressed into the tissue. Tension may be applied to the scaffold to load the tissue and maintain the barbs' grip. The arrangement of the barbs along the scaffold helps to distribute the tension load across a wider area of tissue, rather than concentrating the load at one or more attachment (e.g. suture attachment) points on the tissue, according to embodiments of the present invention. Suture may be used to maintain tension on the scaffold, according to embodiments of the present invention. According to some embodiments of the present invention, suture may be used to tack the scaffold to the surface of the tissue, as a secondary attachment to prevent "walking" (e.g. unintended sliding) of the barbs or loss of contact between the barbs and the tissue, while the primary attachment and load transference occurs via the barbs on the scaffold.

According to some embodiments of the present invention, a rotator cuff tendon is repaired arthroscopically and a barbed scaffold is deployed, optionally sutured to the tendon medially, tensioned, and then anchored to the humerus. The contact of the barbed scaffold with the tendon distributes the load more uniformly across a wider area of the repaired tendon, according to embodiments of the present invention.

Some embodiments of the present invention may include one or more of the following features and/or characteristics in various combinations: (a) barbed structures for attaching reinforcing scaffolds to tissue; (b) scaffolds with multiple barbs to transfer load to tissue; (c) barbed scaffolds with porosity for tissue ingrowth; (d) scaffolds made with barbed fibers; (e) scaffolds or straps that lock upon themselves using barbs that entangle in fibers; (f) scaffolds with regions of barbs for attachment; (g) scaffolds with barbs on just one side of the fabric; (h) braids with barbed fibers in the axial positions; (i) braids with flexible tack strips in the axial positions; (j) braids made with barbed fiber; (k); weaves made with barbed fiber; (I) weaves made with barbed fiber in the warp positions; (m) knits made with barbed fiber; (n) weaves made with flexible tack strips in the weft positions; (o) barbed fiber made from lactide, glycolide, dioxanone, Artelon, hydroxybutyrate and/or resorbable polymers made from other biocompatible monomers; (p) barbed fiber made from biocompatible polymers such as polyesters, nylons, polyaramides, silk, polyacetal, polyetherketones, polyurethanes, and/or polyolefins; (q) flexible tack strips made from resorbable polymers comprising lactides, glycolides, hydroxybutyrates, dioxanones, ε-caprolactone, and/or other biocompatible monomers; (r) flexible tack strips made from biocompatible polymers such as polyesters, nylons, polyaramides, silk, polyacetal, polyetherketones, polyurethanes, and/or polyolefins; (s) braids with barbs in specific regions; (t) scaffolds with barbs on the surface oriented to prevent sliding in one direction; (u) cerclage made with barbs to lock upon itself; (v) a barbed scaffold with stiffness similar to tissue to be repaired so that barbs load the tissue uniformly; (w) barbed scaffolds for simplified arthroscopic delivery and attachment to tissue.

In weaves made with barbed fiber in the warp positions, the barbs on the warp fibers may be at the surface of the weave where they could easily engage other fibers; in such cases, the barbed fiber is flexible enough for a weaving operation, according to embodiments of the present invention. In weaves made with flexible barb strips in the weft positions, the barb strips 600 may be placed into the weft or fill position in such a way that the barb strips 600 do not bend and wrap. Longer barbs may be used in such situations so that the barbs protrude through the warp fibers and still engage tissue or fibers in adjacent materials. The orientation of the barbs, as well as which surface of the fabric from which the barbs protrude, may be controlled by how the barb strips 600 are fed into the loom, according to embodiments of the present invention. Forming a scaffold with barbs protruding from only one side or one surface keeps the barbs internal so as not to irritate tissue, and may have a long contact area with barbs to increase retention strength, according to embodiments of the present invention.

In cerclage or loop devices with barbs that lock the device upon itself, such devices may be resorbable. Resorbable barbs may be used.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. A scaffold (300, 400, 500) for tissue reinforcement comprising a plurality of barbed elements (302, 402, 504, 600) intertwined with a plurality of fibers (304, 404, 502, 804), the plurality of fibers (304, 404, 502, 804) extending along a first longitudinal direction, **characterized in that** the plurality of barbed elements (302, 402, 504, 600) extends along a second longitudinal direction at an angle to the first longitudinal direction.

2. The scaffold according to claim 1, **characterized in that** the plurality of barbed elements is a plurality of barb strips (600) and the plurality of fibers is braided about the plurality of barb strips to form a braided scaffold (500, 800), the plurality of barb strips forming axial components (504, 600) of the braided scaffold.

3. The scaffold according to claim 2, **characterized in that** the plurality of fibers comprises a first set of fibers (810) and a second set of fibers (812), the first set of fibers (810) extending along a first longitudinal direction, the second set of fibers (812) extending along a second longitudinal direction at an angle to the first longitudinal direction, the barb strips (600) extending along a third longitudinal direction at an angle to both the first and second longitudinal directions.

4. The scaffold according to claim 1, **characterized in that** the plurality of barbed elements is a plurality of barb strips (600), and the plurality of barb strips (600) and the plurality of fibers (304, 404, 502, 804) are woven together into a weave, the plurality of barb strips (600) forming weft elements (304, 404) of the weave and the plurality of fibers (302, 402) forming warp elements of the weave.

5. The scaffold according to any of claims 2 or 4, **characterized in that** the plurality of barb strips (600) comprises an average barb length that is at least as long as a diameter of each of the plurality of fibers (304, 404, 502, 804).

6. The scaffold according to any of the preceding claims, **characterized in that** the scaffold further comprises a top surface and a bottom surface, the plurality of barb strips comprising a plurality of barbs (606), each of the plurality of barb strips being oriented within the scaffold such that each of the plurality of barbs (606) extends from the bottom surface but not the top surface.

7. The scaffold according to any of claims 2 to 6, **characterized in that** each of the plurality of barb strips (600) comprises a resorbable polymer comprising one or more materials selected from the group: lactides, glycolides, hydroxybutyrates, ε-caprolactone, and dioxanones.

8. The scaffold according to any of claims 2 to 6, **characterized in that** each of the plurality of barb strips (600) comprises a biocompatible polymer comprising one or more materials selected from the group: polyesters, nylons, polyaramides, silk, polyacetal, polyetherketones, polyurethanes, and polyolefins.

9. The scaffold according to any of claims 2 to 8, **characterized in that** the plurality of barb strips (600) is flexible.

10. The scaffold according to any of the preceding claims, **characterized in that** the plurality of fibers is a plurality of non-barbed fibers (412, 502, 804).

11. The scaffold according to any of the preceding claims, **characterized in that** the angle is a right angle.

## Amended claims

### Amended claims in accordance with Rule 137(2) EPC.

**1.** A scaffold (300 ; 400 ; 500 ; 800 ; 902 ; 1002) for tissue reinforcement, comprising a plurality of fibers (304 ; 404 ; 502 ; 804) extending along a first longitudinal direction, **characterized in that** the scaffold (300, 400, 500 ; 800 ; 902 ; 1002) further comprises plurality of barb strips (302, 402, 504, 600) intertwined with the plurality of fibers (304, 404, 502, 804) and extending along a second longitudinal direction at an angle to the first longitudinal direction.

**2.** The scaffold (500 ; 902 ; 1002) according to claim 1, **characterized in that** the plurality of fibers (502 ; 804) is braided about the plurality of barb strips (504 ; 600) to form a braided scaffold (500 ; 800 ; 902 ; 1002), the plurality of barb strips (504 ; 600) forming axial components (504, 600) of the braided scaffold.

**3.** The scaffold (500 ; 902 ; 1002) according to claim 2, **characterized in that** the plurality of fibers (804) comprises a first set of fibers (810) and a second set of fibers (812), the first set of fibers (810) extending along a first longitudinal direction, the second set of fibers (812) extending along a second longitudinal direction at an angle to the first longitudinal: direction, the barb strips (600) extending along a third longitudinal direction at an angle to both the first and second longitudinal directions.

**4.** The scaffold (300 ; 400 ; 902 ; 1002) according to claim 1, **characterized in that** the plurality of barb strips (600) and the plurality of fibers (304 ; 404) are woven together into a weave, the plurality of barb strips (600) forming weft elements (304, 404) of the weave and the plurality of fibers (302, 402) forming warp elements of the weave.

**5.** The scaffold (300 ; 400 ; 500 ; 800 ; 902 ; 1002) according to any of claims 2 or 4, **characterized in that** the plurality of barb strips (302 ; 402 ; 504 ; 600) comprises an average barb length that is at least as long as a diameter of each of the plurality of fibers (304, 404, 502, 804).

**6.** The scaffold (400) according to any of the preceding claims, **characterized in that** the scaffold (400) further comprises a top surface and a bottom surface, the plurality of barb strips (402 ; 600) comprising a plurality of barbs (406 ; 606), each of the plurality of barb strips (402 ; 600) being oriented within the scaffold such that each of the plurality of barbs (406 ; 606) extends from the bottom surface but not the top surface.

**7.** The scaffold (300 ; 400 ; 500 ; 800 ; 902 ; 1002) according to any of the preceding claims, **characterized in that** each of the plurality of barb strips (302 ; 402 ; 504 ; 600) comprises a resorbable polymer comprising one or more materials selected from the group: lactides, glycolides, hydroxybutyrates, ε-caprolactone, and dioxanones.

**8.** The scaffold (300 ; 400 ; 500 ; 800 ; 902 ; 1002) according to any of claims 1 to 16, **characterized in that** each of the plurality of barb strips (302 ; 402 ; 504 ; 600) comprises a biocompatible polymer comprising one or more materials selected from the group: polyesters, nylons, polyaramides, silk, polyacetal, polyetherketones, polyurethanes, and polyolefins.

**9.** The scaffold (300 ; 400 ; 500 ; 800 ; 902 ; 1002) according to any of claims 1 to 6, **characterized in that** the plurality of barb strips (302 ; 402 ; 504 ; 600) is flexible.

**10.** The scaffold (300 ; 400 ; 500 ; 800 ; 902 ; 1002) according to any of the preceding claims, **characterized in that** the plurality of fibers (304 ; 404 ; 502 ; 804) is a plurality of non-barbed fibers.

**11.** The scaffold (300 ; 400 ; 902 ; 1002) according to any of the preceding claims, **characterized in that** the angle is a right angle.
